# EUROPEAN PATENT SPECIFICATION

(11) **EP 0 658 118 B1**
(45) Date of publication and mention of the grant of the patent: **23.01.2002**
(21) Application number: 93921251.0
(22) Date of filing: 30.08.1993
(51) Int. Cl.: A61K 39/095, A61K 39/385, A61K 39/40, C07H 1/00

(54) **VACCINES AGAINST GROUP C NEISSERIA MENINGITIDIS**
IMPFSTOFFE GEGEN NEISSERIA MENINGITIDIS GRUPPE C
VACCINS CONTRE LA NEISSERIA MENINGITIDIS DU GROUPE C

(30) Priority: 31.08.1992 US 938367; 19.05.1993 US 64501
(43) Date of publication of application: 21.06.1995
(73) Proprietor: Baxter Healthcare S.A., 8306 Wallisellen (CH)
(72) Inventor: MICHON, Francis, Bethesda, MD 20814 (US); JENNINGS, Harold, Gloucester, Ontario, Canada K1J6 G6 (CA); TAI, Joseph Y., Pennsylvania 19034 (US); HRONOWSKI, Lucjan J.J., Maryland 20723 (US); MATES, Sharon, Chevy Chase, Maryland 20815 (US)
(74) Representative: Laufhütte, Dieter, Dr.-Ing.
(86) International application number: US9308155
(87) International publication number: WO9405325

(56) References cited:
- WO-A-93/07178
- US-A- 5 097 020
- VACCINE, vol. 10, no. 10, 1992 GUILDFORD GB, pages 691-698, COSTANTINO P. ET AL. 'Development and phase 1 clinical testing of a conjugate vaccine against meningococcus A and C'
- DEVELOPMENTS IN BIOLOGICAL STANDARDIZATION, vol. 65, 1986 pages 197-204, BEUVERY E.C. ET AL. 'Characteristics of two types of meningococcal group C polysaccharide conjugates using Tetanus Toxoid as carrier protein'
- The Journal of Immunology, Volume 127, No. 3, issued September 1981, JENNINGS et al., "Immunochemistry of Groups A, B, and C Meningococcal Polysaccharide-Tetanus Toxoid Conjugates", pages 1011-1017, see entire document, especially page 1014.
- Clinical Microbiology Reviews, Volume 2, Supplement, issued April 1989, FRASCH, "Vaccines for Prevention of Meningococcal Disease", pages s134-s138, see pages s134-s135.
- Microbial Pathogenesis, Volume 6, issued 1989, ASHTON et al., "Protective Efficacy of Mouse Serum to the N-Propionyl Derivative of Meningococcal Group B Polysaccharide", pages 455-458, see entire document.
- Protective Groups in Organic Synthesis, Second Edition, by Theodora Greene and Peter Wuts, p. 406-421
- Journal Infectious Disease, 139(1), 1979, 52-59
- Infection and Immunity, 34(1), 191, 144-146

## Description

### FIELD OF THE INVENTION

The present invention relates to the preparation of vaccines useful against group C Neisseria meningitidis wherein these vaccines comprise conjugates of modified group C capsular polysaccharides.

### BACKGROUND OF THE INVENTION

Group C Neisseria meningitidis is the cause of a large percentage of bacterial meningitidis worldwide. Current measures to prevent this disease consist of vaccines composed of purified group C capsular polysaccharide. These vaccines are effective in adults but are poorly immunogenic in children. The poor results in infants is highly undesirable since this section of the population has the highest incidence of these infections.

The group C meningococcal polysaccharide (GCMP) is a weak immunogen and, hence, there have been efforts to enhance the immunogenicity of these polysaccharides to expand their usefulness as vaccines. One possible method of achieving this goal, which has shown some promise is by conjugation of these polysaccharides to a carrier, such as a protein.

Various investigators have isolated and purified intact capsular polysaccharides and have covalently coupled them to carrier proteins. These conjugates are more immunogenic than the polysaccharide alone. Many examples can be found in the literature which illustrate the success of these conjugate antigens. One such example, U.S. Pat. No. 4,673,574 describes the use of bacterial capsular polysaccharides conjugated to carrier proteins to form immunogenic compounds. Another example, U.S. Pat. No. 4,356,170 describes the immunogenicity of conjugates consisting of a specific polysaccharide, group A meningococcal polysaccharide (GAMP) coupled to a carrier protein. These conjugates provide an improved method for delivering effective levels of antigen to a host.

Another method for increasing immunogenicity has shown promise with the group B meningococcal polysaccharide (GBMP) antigens, as seen in U.S. Pat. No. 4,727,136. This method entails the replacement of N-acetyl groups of the sialyl moiety of the polysaccharide with N-propenyl groups followed by conjugation of the modified polysaccharide to a carrier protein. These conjugates are good immunogens and effectively cross-react with the native GBMP. This finding demonstrates that the addition of substituents onto the polysaccharide antigen can create additional immunogenic sites or epitopes for recognition by antibodies.

Vaccines which are currently prepared from O-acetyl-positive group C N. meningitidis are known to be poorly immunogenic. There have been reports that vaccine derived from an O-acetyl-negative variant is immunogenic. See Glode et al. The Journal of Infectious Disease Vol. 139, No. 1, Jan. 1979 pp. 52-59; Steinkoff et al., Infection and Immunity, Oct. 1981 pp. 144-145; and Arakere et al., Infection and Immunity, Dec. 1991, pp. 4349-4356.

Most group C N. meningitidis strains produce an O-acetyl positive (OAc+) polysaccharide in which the O-acetyl groups are distributed exclusively between C-7 and C-8 of its sialic acid residues. Amongst the known OAc-negative (OAc-) strains is N. meningitidis group C MC 19 bacteria.

It is an object of the present invention to provide a highly immunogenic vaccine comprising a modified group C meningitidis polysaccharide conjugated to a carrier and also to provide a highly immunogenic vaccine containing the conjugate of a carrier and a modified polysaccharide derived from a OAc negative variant.

### SUMMARY OF THE INVENTION

The present invention relates to the chemical modification by O-deacetylation of the Neisseria meningitidis group C polysaccharide (GCMP) derived from OAc+ strains and the conjugation of the deacetylated polysaccharide to a carrier to provide an enhanced immunogenic material.

The invention further relates to a method of fragmenting the deacetylated polysaccharide from OAc+ strains and also fragmenting polysaccharide from OAc-strains in order to decrease the effective size of these polysaccharides.

The invention further relates to the depolymerization of group C polysaccharide which is either native or deacetylated to provide lower molecular weight fragments having enhanced immunogenic activity.

The invention also relates to a method of creating a residue within the deacetylated polysaccharide from OAc+ strains which provides a reactive site for coupling either directly to a carrier material or to carrier material via a linking molecule.

In addition, this invention relates to an immunogenic conjugate produced by coupling an O-deacetylated meningococcal C polysaccharide, a polysaccharide from an OAc- strain to a carrier material or a fragmented meningococcal C polysaccharide, wherein the polysaccharide and carrier are covalently coupled.

In the present invention, a method is disclosed of preparing immunogenic conjugates comprising covalently coupling a carrier material and a O-deacetylated group C polysaccharide of Neisseria meningitidis or a polysaccharide form an OAc- strain or fragments of these polysaccharides or fragments of non-deacetylated polysaccharide.

This invention further relates to the use of the conjugate of the O-deacetylated group C polysaccharide or an OAc- polysaccharide as a potent bactericidal vaccine in mammals susceptible to bacterial meningitidis. The vaccine may include an adjuvant such as aluminum hydroxide or stearly tyrosine.

### DETAILED DESCRIPTION OF THE INVENTION

In order to enhance the immunogenicity of the group C meningococcal polysaccharide (GCMP), a modified GCMP is generated and covalently coupled to a carrier molecule. Additionally, polysaccharide from an OAc-strain either as isolated or in modified form is covalently coupled to a carrier molecule. The polysaccharide conjugate can be formed from a variety of chemical, physical or enzymatic coupling reactions. The coupling technique used is dependent upon the linking agents which might be employed and the reactive groups on the carrier material or molecule. A variety of coupling procedures are known to the skilled artisan and could be readily employed.

The carrier material or molecule is defined herein as any material or molecule having groups capable of being coupled to polysaccharide moieties. Polymeric carrier can be a natural or a synthetic material, and may be water soluble or water insoluble. Carrier materials or molecules are most commonly proteinaceous in nature. In the case of a protein carrier, the protein can be any physiologically tolerated protein having a reactive group available for coupling. Any type of carrier may be used, which contains a primary or a secondary amino group. The reactive free amino groups can conveniently be coupled to polysaccharide via moieties, such as hydroxyls, aldehydes, carboxylic acids and the like. Suitable proteins include natural peptides and proteins, such as bovine serum albumin, bacterial toxoids derived from bacterial toxins by their synthetic modification, e.g. diphtheria toxoid, tetanus toxoid, Pertussis toxin or Pertussis toxoid, Pseudomonas aeruginosa recombinant exoprotein A and Clostridium perfringens exotoxins. Other proteins derived from bacteria may also be employed. The bacterial source may be, for example, Hemophilus influenza, meningococci, pneumococci β-hemolytic streptococci and E. Coli. Other proteins containing lysine residues, such as a synthetic polylysine may also be useful. Other protein carriers as well as other non-proteinaceous carrier molecules are readily known to those skilled in the art and could be used as a carrier molecule in the present invention. Examples of water insoluble carriers are aminoalkyl-Sepharoses, e.g. aminopropyl or aminohexyl Sepharose, and aminopropyl-glass. Other carriers may also be used which are modified to contain a chemically linked amino group. Such carriers may be derived from polysaccharides to which an aminoalkyl group is attached through a covalent linkage.

According to one aspect of the present invention, the O-acetyl groups on positions 7 and/or 8 of the sialyl moieties in the group C polysaccharide from OAc+ strains are selectively removed to a varying extent from the meningococcal group C polysaccharide by treatment with an appropriate reagent. Many deacetylating reagents are known to those skilled in the art. Preferably, the deacetylation is carried out under mild basic conditions in aqueous medium, such as, in about 0.01 to 0.5 N sodium hydroxide, preferably 0.1 N sodium hydroxide at about 0 to 50°C, preferably 25°C. Other basic solutions, such as potassium hydroxide, ammonium hydroxide and the like, can also be used for this reaction. The deacetylation reaction temperature and base concentration used in the reaction can be varied keeping in mind only that mild condition should be maintained. It is preferred that the reaction conditions should be controlled such that only deacetylation of the group C polysaccharide is achieved to the desired extent. The O-deacetylation is carried out until the desired degree of deacetylation is achieved, i.e., for about 1 to 25 hours, preferably for about 16 hours in 0.1 N NaOH.

The yield of the O-deacetylated polysaccharide from the deacetylation reaction using mild conditions is typically in excess of 80%. The degree of O-deacetylation may vary from about 30% to 100% depending on the conditions employed. Typically, varying the temperature and reaction time allows one to vary the degree of O-deacetylation on the polysaccharide. It is preferred that O-deacetylation in the range of greater than 80% and most preferably 100% deacetylation is achieved.

It may also be desirable to fragment or depolymerize the polysaccharide chain (before or after deacetylation) prior to conjugation to the carrier molecule. Polysaccharides are sensitive to many reagents and may be hydrolyzed with weak acids such as acetic acid or oxidized by reagents such as metaperiodate. In a preferred embodiment of the present invention, non-deacetylated polysaccharide deacetylated polysaccharide or polysaccharide from OAc- strains is both fragmented and activated for conjugation by metaperiodate oxidation. Those skilled in the art would recognize other methods for accomplishing this step of the reaction.

The immunogenic conjugates of the invention are prepared by fist forming reducing end groups on fragments of the O-deacetylated GCMP or the polysaccharide from OAc-strains and then reacting the reducing end groups with amine groups of the carrier material or molecule by means of reductive amination. The reducing end groups may be formed by any suitable method, including selective hydrolysis, i.e., by acids or enzymes, or by oxidative cleavage, i.e., by metaperiodate. The conjugation is preferably achieved by reductive amination in an aqueous solution containing a reducing agent such as cyanoborohydride anions. Other methods for coupling a polysaccharide to a carrier molecule are known to one skilled in the art.

A preferred method for coupling involves a two step reaction. The polysaccharide is metaperiodate oxidized to create aldehyde moieties and is then reacted in the presence of free amino groups on the carrier molecule and in the presence of a suitable reducing agent. Suitable reducing agents would typically include cyanoborohydride, sodium borohydride or another reducing agent which will not reduce the reducing ends of interest nor adversely affect the carrier molecule or material. The reducing agent acts as a mild selective reducing agent of the Schiff base intermediate formed between the carbonyl groups of the hydrolyzed GCMP fragment and the amino groups of the carrier molecule or material. Typical reaction conditions for this type of conjugation are given in the Jennings et al. Patent U.S. 4,356,170.

It is to be understood, however, that the conjugate vaccines of the invention are not limited to those produced via reductive amination. Thus, the vaccines may also be produced by conjugating the polysaccharide with the carrier protein using an adipic dihydrazide spacer, as described by Schneerson, R., et al., Preparation, Characterization and Immunogenicity of Haemophilus Influenzae Type b Polysaccharide-Protein Conjugates, J. Exp. Med., 1952, 361-476 (1980), and in U.S. Patent 4,644,059 to Lance K. Gordon. Alternatively, there may be used the binary spacer technology as described by Marburg, S., et al., "Biomolecular Chemistry of Macromolecules: Synthesis of Bacterial Polysaccharide Conjugates with Neisseria meningitides Membrane Protein", J. Am. Chem. Soc., 108, 5282-5287 (1986) or, possibly, the reducing ends methodology, as referred to by Anderson in U.S. patent 4,673,574.

The immunogenic conjugates of the invention may be formulated with one or more pharmaceutically acceptable excipients and optionally other ingredients. The pharmaceutically acceptable excipient(s) must be "acceptable" in the sense of being compatible with the other ingredients of the formulation and not deleterious to the recipient thereof. The formulations may conveniently be presented in u;nit dosage form and may be prepared by any method well-known in the pharmaceutical art.

Preparation of vaccines which contain polysaccharide conjugates as active ingredients is well understood in the art. Typically, such vaccines are prepared as injectables either as liquid solution or suspensions; solid forms suitable for solution in, or suspension in, liquid prior to injection may also be prepared. The preparation may also be emulsified. The active immunogenic ingredient is often mixed with excipient which are pharmaceutically acceptable and compatible with the active ingredient. Suitable excipient are, for example, water, saline, dextrose, glycerol, ethanol, or the like and combinations thereof. In addition, if desired, the vaccine may contain minor amounts of auxiliary substances such as wetting or emulsifying agents, pH buffering agents, or adjuvants which enhance the effectiveness of the vaccine. Any suitable adjuvant known to those skilled in the art may be used. Suitable adjuvants include inter alia, stearyl tyrosine, aluminum hydroxide, aluminum phosphate and aluminum sulfate. It has been found that vaccines made with aluminum hydroxide adjuvant are especially effective. The vaccines are conventionally administered parenterally, by injection, for example, either subcutaneously or intramuscularly. Additional formulations which are suitable for other modes of administration include suppositories and, in some case, oral formulations. For suppositories, traditional binders and carriers may include, for example polyalkalene glycols or triglycerides; such suppositories may be formed from mixtures containing the active ingredient in the range of 0.5% to 10%, preferably 1-2%. Oral formulations include such normally employed excipients as, for example,pharmaceutical grades of mannitol, cellulose, magnesium carbonate and the like. These compositions take the form of solutions, suspensions, tablets, pills, capsules, sustained release formulations or powders and contain 10%-95% of active ingredient, preferably 25-70%.

The vaccines are administered in a manner compatible with dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practitioner and are peculiar to each individual. However, suitable dosage ranges are of the order of several hundred micrograms active ingredient per individual. *Suitable regimes* for initial administration and booster shots are also variable, but are typified by an initial administration followed in one or two week intervals by a subsequent injection or other administration.

The vaccine of the present invention elicits effective levels of anti-GCMP antibodies in mammals. This vaccine may be administered by injection to mammals of any age and may be utilized to induce active immunization against systemic infection caused by *Neisseria meningitidis* by administering an immunogenic amount of the conjugate mammals susceptible to the disease.

Generally, vaccines containing from about 5 to about 100 µg, preferably about 10 to 50 µg, are suitable to elicit effective levels of antibody against GCMP in young mammals. The exact dosage would be determined by experimentation. Several small doses given sequentially would be expected to be superior to the same amount of conjugate given as a single injection.

In yet another aspect, the invention provides a gamma globulin fraction capable of protection against meningitidis caused by group C *N meningitidis*. The fraction is produced by immunizing a mammal with the vaccine of the present invention. The fraction is then administered to an individual to provide protection against or to treat on-going infection caused by the above organisms. From this, it will be appreciated that the immunogenic vaccine conjugates of the invention will provide for a source of therapeutic antiserum in light of their favorable immunogenicity. The conjugates of the invention will also be useful for raising monoclonal antibodies and, possibly, anti-idiotype antibodies.

O-Deacetylation of the meningococcal group C polysaccharide can be carried out by treatment with mild base such as, 0.01 to 0.5 N NaOH preferably 0.1 N NaOH at about 0 to 50°C, preferably 25°C for about 1 to 25 hours, preferably about 16 hours. The removal of the acetyl groups from the 7 and/or 8 positions is verified by NMR spectroscopy.

In the subsequent steps the polysaccharide is purified by gel filtration such as, on Superdex 200 prep grade. Reductive partial depolymerization using sodium periodate is carried out on the non-deacetylated polysaccharide, the deacetylated polysaccharide form OAc+ strains and the polysaccharide from OAc- strains to afford an activated polysaccharide having the optimal size for eliciting the maximal immune response. The polysaccharide is purified by gel filtration, dialyzed to remove salts and lyophilized. It is then coupled to an appropriate protein carrier such as tetanus toxoid by the reductive amination reaction. The resulting conjugates are purified by gel filtration and analyzed for immunogenicity in FRW outbred Swiss Webster mice purchased from Charles Rivers.

### BRIEF DESCRIPTION OF THE FIGURES

Fig. 1. The effect of polysaccharide O-acetylation in the GCMP-TT conjugates on the serum ELISA titers. The ELISA plates were coated with O-acetyl negative GCMP-HSA conjugate.

Fig. 2. The effect of polysaccharide O-acetylation in the GCMP-TT conjugates on the serum ELISA titers. The ELISA plates were coated with O-acetyl positive GCMP-BSA conjugate.

Fig. 3. The effect of polysaccharide O-acetylation in the GCMP-TT conjugates on the complement dependent bactericidal activity. The bactericidal activity was determined against *N meningitidis* group C strain C 11, a O-acetyl positive strain.

Fig. 4. The effect of polysaccharide size in the GCMP-TT conjugates on the serum ELISA titers. The ELISA plates were coated with O-acetyl negative GCMP-HSA conjugate.

Fig. 5. The effect of polysaccharide size in the GCMP-TT conjugates on the serum ELISA titers. The ELISA plates were coated with O-acetyl positive GCMP-BSA conjugate.

Fig. 6. The effect of polysaccharide size in the GCMP-TT conjugates on complement dependent bactericidal activities. Bactericidal activity was determined against *N meningitidis* group C strain C11, a O-acetyl positive strain.

### Example 1

### O-Deacetylation of Group C Meningococcal Polysaccharide (GCMP)

Native meningococcal group C polysaccharide (188 mg) was dissolved in 0.1 N sodium hydroxide solution (30 ml). The mixture was stirred at 25°C for 16 hrs. It was then dialyzed at 4°C against water and lyophilized. The polysaccharide (144 mg, 77%) which was obtained as a white cottony solid was shown ¹H-N.M.R. spectroscopy (500 MHz) to be *O*-deacetylated.

### Example 2

### Purification of the O-Deacetylated Group C Polysaccharide (GCMP) - (-)

The O-deacetylated polysaccharide (141 mg) was dissolved in Dulbecco's PBS (10 mL) and purified by gel filtration on a Superdex 200 prep grade (2.6 X 100 cm) column. The column was eluted at 100 ml/hr and 5.0 ml fractions were collected. The eluate was monitored with a differential refractometer and a UV (280 nm) detector. The fractions containing the first major peak detected with the differential refractometer and which were substantially free of UV absorbing material were combined. After dialysis against water and lyophilization purified GCMP-(-) (108 mg. 77%) was obtained as a white cottony solid. The solid material was analyzed for protein by the Bradford method and for nucleic acids by the UV absorbance at 260 nm. As shown in Table 1, these contaminants are substantially reduced by this purification step. In addition analysis of GCMP-(-) by HPLC using the Superose 12 gel filtration column (Pharmacia) showed that it elutes as a sharp void volume peak as it was also observed for the starting native polysaccharide (GCMP) indicating that the purified polysaccharide is present in a high molecular weight form.

### Example 3

### Oxidation and Sizing of Purified GCMP-(-)

The oxidation of the polysaccharide with sodium metaperiodate serves the dual purpose of activating the polysaccharide for subsequent coupling to proteins and to cleave the polysaccharide into smaller fragments which have been shown previously to give the optimal immune response. Since it has been also shown previously that different lots of polysaccharide require different periodate oxidation conditions in order to achieve the optimal polysaccharide chain lengths these conditions are first determined on small scale reactions (5 mg) after which the large scale reaction is performed. Typically the reaction time and temperature is kept constant at 2 hours and 25°C respectively and the concentration of the sodium metaperiodate is varied between 3 and 12 millimolar in deionized water. The concentration of the polysaccharide in the small and large scale reactions is also kept constant at 6-10 mg/ml. At the end of the 2 hour period at an excess (-10-fold) of a 1.0 M ethylene glycol solution is added to the reaction solution in order to destroy unreacted sodium metaperiodate. Following at least 30 minutes of reaction the solutions are analyzed by HPLC using a calibrated Superose-12 column in order to determine the average molecular weight of the oxidized polysaccharide. The reaction conditions which provide the polysaccharide having an average of molecular weight of 12,000-16,000 daltons are used in the large scale reactions as illustrated by the following reaction.

Purified GCMP-(-) (63 mg) was dissolved in deionized water (4.81 ml). To this solution was added a 21 mmol sodium metaperiodate solution (4.81 mL) to give a reaction mixture in deionized water having the polysaccharide at 6.55 mg/mL and the NaI04 at 10.5 mmol. The reaction was allowed to proceed at 25°C for 2 hours in the dark and then treated with 1 M ethylene glycol (0.96 Ml) for 30 min. This material elutes on the Superose-12 column as a broad peak having an average molecular weight of 12,800 daltons. The volume of the solution was reduced to about 5 ml on the lyophilizer and the material was purified by gel filtration on a Superdex 200 prep grade column (2.6 X 100 cm) using Dulbecco's PBS as the eluting buffer and 5 mL fractions were collected. Those fractions which contained material having an average molecular weight between 10,000-18,0-00 are combined. After dialysis against deionized water and lyophilization the oxidized and sized polysaccharide [o]-GCMP-(-) (16.0 mg, 23%) was obtained as a white cottony solid which was shown by FPLC on Superose-12 to have an average molecular weight of 14,500 daltons.

**Table 1**

| Protein and Nucleic Acid Contents of Group C Meningococcal Polysaccharide Preparations | | |
|---|---|---|
| Polysaccharide | % Protein | % Nucleic Acids |
| GCMP | 8.3 | 2.7 |
| Purified GCMP-(-) | 0.61 | 0.12 |
| [o]-GCMP-(-) | 0.06 | 0.31 |

### Example 4

### Preparation of [o]-GCMP-(+)

Native GCMP (350 mg) was dissolved in 0.02 N NaOH (36 mL) and incubated at 25°C for 3 hours. To this solution was then added 0.1 M NaIO₄ (36 mL) and the contents were incubated at 25°C a further 50 min. The reaction was then treated with 50% ethylene glycol (5.3 mL) for 20 min. and the mixture was clarified by centrifugation at 5000 rpm for 10 min. The supernatant was dialyzed against deionized water and lyophilized to afford 300 mg of solid. The material was sized on Bio-Gel A-0.5 m column to provide the oxidized and sized polysaccharide {[o]-GCMP-(+)} having a molecular weight of 11,500 daltons.

### Example 5

### Purification of the tetanus toxoid protein monomer (TT)

Into a gently stirred tetanus toxoid solution (Statens Seruminstitu) [containing 2.1 mg/mL of protein (Bradford protein assay using human IgG as standard)] (75 mL) was added in small portions over a period of 45 min ammonium sulfate (42.1 g) to give an 80% saturated solution. The turbid solution was left at 4°C overnight to complete the precipitation of the protein. The precipitate was collected by centrifugation at 15,000 rpm (23,000 g) for 20 min and the supernatant which contained less than 10% of the original protein was discarded. The pellet was redissolved in Dulbecco's PBS buffer (10 mL) and fractionated by gel filtration on a Bio-Gel A-0.5 m column. The major peak corresponding to the tetanus toxoid monomer was pooled dialyzed against deionized water and lyophilized to give TT as a white cottony solid (65.4 mg, 41%).

### Example 6

### Coupling of O-deacetylated group c meningococcal polysaccharide {[o]-GCMP-(-)} to tetanus toxoid (TT)

A solution containing [o]-GCMP-(-) (10 mg), Tt (3.3 mg) and sodium cyanoborohydride (6.8 mg) in 0.20 M phosphate buffer pH 7.5 (0.222 mL) was kept at 37°C for days . The reaction solution was diluted with PBS buffer (0.5 mL) and insoluble matter was removed by centrifugation in an Eppendorf centrifuge at 10,000 rpm for 2 min. The supernatant was loaded unto a 1.6 X 100 cm Bio-Gel A-0.5 m column and eluted with 0.15 M NaCl + 0.02% thimerosal at 20 mL/hr. Fractions corresponding to the peak eluting at the void volume were pooled to afford the O-deacetylated group C meningococcal polysaccharide-tetanus toxoid conjugate [GCMP-(-)-TT]. The pooled solution was analyzed for protein by the Bradford method and shown to contain 2.86 mg of protein (87% yield). The polysaccharide content in the conjugate was estimated from the content of sialic acid, which was determined by the resorcinol assay for sialic acid, and was shown to contain 0.997 mg of polysaccharide (10% yield). The characteristics of the conjugate are summarized in Table 2.

### Example 7

### Coupling of O-Acetylated group C meningococcal polysaccharide {[o]-GCMP-(+)} to tetanus toxoid

A solution containing [o]-GCMP-(+) (10.2 mg), TT (2.9 mg) and sodium cyanoborohydride (5.2 mg) in 0.20 M phosphate buffer pH 7.5 (0.20 mL) was kept at 37°C for 5 days. The conjugate was purified y gel filtration chromatography and analyzed as described in Example 6. This conjugate solution contains 2.7 mg (93% yield) of protein and 1.06 mg (10.4% yield) of polysaccharide. The characteristics of this conjugate are summarized in Table 2.

**Table 2**

| Characteristics of the GCMP-(-)-TT and GCMP-(+)-TT conjugates | | | | |
|---|---|---|---|---|
| Conjugate | Polysaccharide | | Protein: PS | % Polysaccharide in the conjugate |
| | Acetylation | Average Mol. Wt. | | |
| GCMP-(-)-TT | O-Deacetylated | 12,500 | 2.9:1 | 26 |
| GCMP-(+)-TT | O-Acetylated | 11,500 | 2.6:1 | 28 |

Example 7A

### Preparation of (-)-GCMP-TT Vaccine

The (-)-GCMP-TT vaccines are prepared in the same manner as the GCMP-(-)-TT vaccines in the above examples except that the polysaccharide is obtained from N. meningitidis group C strain MC 19 bacteria. This bacteria produces GCMP which lacks the O-acetyl moieties.

### Example 8

### Preparation of the conjugate vaccine solutions and biological studies

The conjugate solutions described in Examples 6 and 7 were diluted with saline (0.15 M NaCl + 0.02% thimerosal) such that the concentration of polysaccharide in all of the vaccines is 10 µg/mL. Half of these vaccines also contained the aluminum hydroxide adjuvant at a concentration of 1.0 mg/mL.

The immunogenicities of these vaccines were assayed in 4-6 week old outbred Swiss Webster CFW female mice. The animals were injected subcutaneously with 0.2 mL of the vaccines on day O, 14 and 28 and the sera for antibody assay were collected on day 38.

### Example 9

### Enzyme-linked immunosorbant assay (ELISA) for mouse antibodies to Neisseria meningitidis group C polysaccharide

The ELISA was performed by coating microtiter plates with the appropriate polysaccharide [GCMP-(-) OR GMCP-(+)] human serum albumin (HSA) conjugate followed by treatment with BSA blocking buffer. Dilutions of the sera in PBS-Tween buffer were then applied. The plates were incubated at room temperature for 1 hour, washed and treated with the second antibody [goat antimouse IgG (H+L) - peroxidase] at room temperature for 30 minutes. This was followed by thorough washing with PBS-Tween and treatment with freshly prepared TMB peroxidase substrate, as described in Kirkegaard & Perry (please insert citation). After 15 minutes, the enzyme reaction was stopped with 1 M phosphoric acid added to each well, and the absorbance was analyzed using a microplate reader. The ELISA titers are summarized in Table 3.

### Example 10

### Bactericidal assay for Neisseria meningitidis serogroup C

The bactericidal activity was assayed by incubating Neisseria meningitidis group C (strain C11) in the presence of an appropriate dilution of the test sera and baby rabbit serum (complement source) at 37°C for 1 hr. Two negative controls, in one case lacking the test sera and in the second case containing heat inactivated complement were similarly incubated. Aliquots of these solutions (5µl) were then applied onto chocolate agar plates in duplicate and incubated at 35-37°C in 5% CO₂ overnight in order to assay for survivors. The next morning the number of colonies are counted on each plate and averages calculated for the duplicates. The serum bactericidal titer is reported as the serum dilution yielding 50% survival. The bactericidal activities for the different vaccines are summarized in Table 3

**Table 3**

| Vaccine | | ELISA titer | Bactericidal titer |
|---|---|---|---|
| GCMP-(+) | in Saline | 230 | No Response |
| GCMP-(+)-TT | in Saline | 12,293 | No Response |
| GCMP-(+)-TT | in Saline and Alum | 27,866 | 2,800 |
| GCMP-(-)-TT | in Saline | 13,127 | 2,700 |
| GCMP-(-)-TT | in Saline and Alum | 25,537 | 21,000 |
| (-)-GCMP-TT | Saline | 10,867 | 2,700 |
| (-)-GCMP-TT | Alum | 27,327 | >40K |

Analysis of the sera obtained from groups of mice immunized with the two types of conjugate by ELISA revealed the presence of high titers of IgG antibody in both groups of mice. Further analysis of the sera using a bactericidal assay against the modified strain revealed a surprising result. While the ELISA titer for sera obtained from both vaccine groups were similar, the sera obtained from mice immunized with the modified conjugate had superior levels of bactericidal antibodies when compared to sera from mice that had received the native conjugate vaccine. The results of this study suggest that the present conjugate vaccine elicit a class of antibodies that have potent bactericidal activity.

### Synthesis of the Group C meningococcal Polysaccharide-Tetanus Toxoid Conjugates (GCMP-TT)

Group C meningococcal polysaccharide isolated from O-acetylpositive strains was purified and partially or completely O-deacetylated with dilute base. The O-deacetylated polysaccharide as well as purified polysaccharide lots not treated with base were partially depolymerized and activated and purified. The extent and position of O-acetylation on the purified and activated polysaccharide lots were determined by high resolution Nuclear Magnetic Resonance (NMR) SPECTROSCOPY (600 MHz) and the average molecular weight by gel filtration column chromatography. See Table 4 for a summary of this data. Coupling (4) of these activated polysaccharides to tetanus toxoid protein afforded the GCMP-TT conjugates shown in Table 5 which are also summarized the % polysaccharide contents in the GCMP-TT conjugates.

### Preparation of Vaccine Solutions and Biological Studies

The GCMP-TT conjugate solutions were diluted with saline containing 0.01% thimerosal and used directly for immunizing mice (Saline) or adsorbed on aluminum hydroxide (Al Adjuvant) or stearyl tyrosine (St Adjuvant). Each final vaccine solution or mixture contained the conjugate at 10 µg/ml in terms of the conjugate polysaccharide content. All vaccine formulations along with tetanus toxoid and polysaccharide controls were tested in groups of 10 female mice (4-6 weeks old) by subcutaneous injection of 0.2 ml of the vaccine solutions or mixtures on days 0, 14 and 28. The sera was collected on day 38 and stored frozen until it was analyzed by the ELISA or bactericidal assay.

### Serological Studies

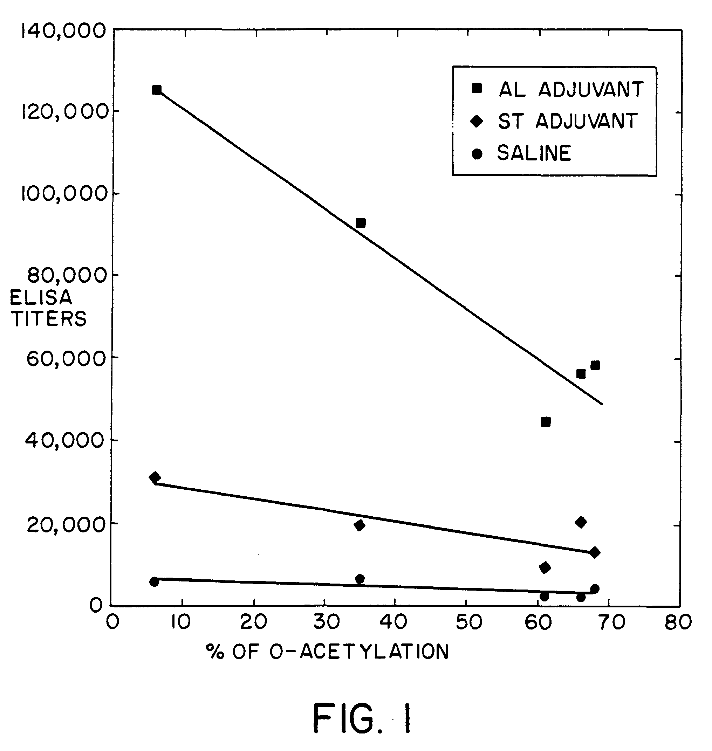

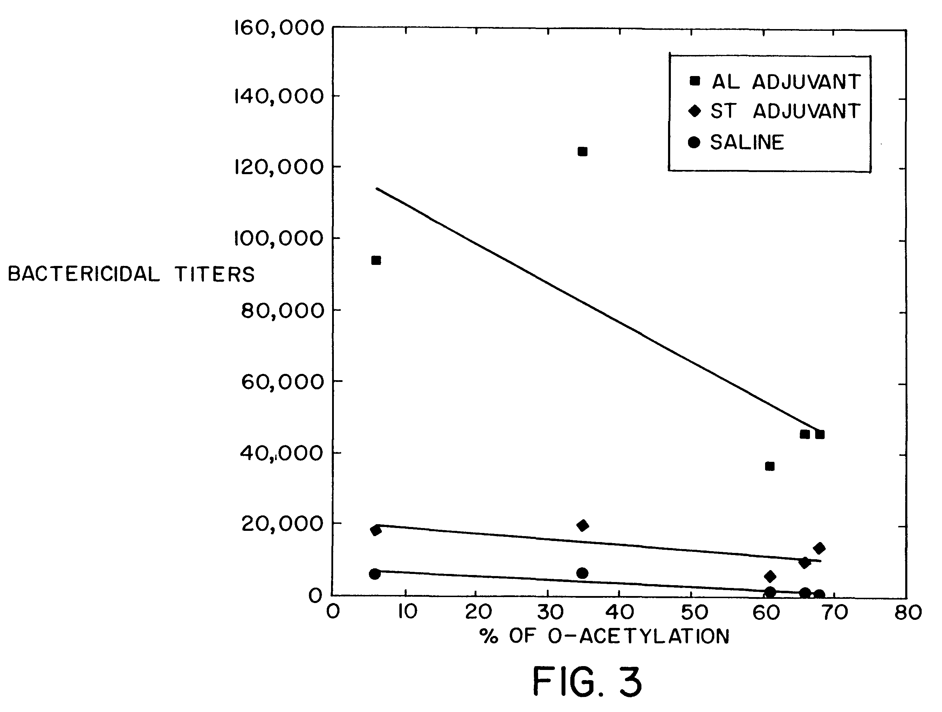

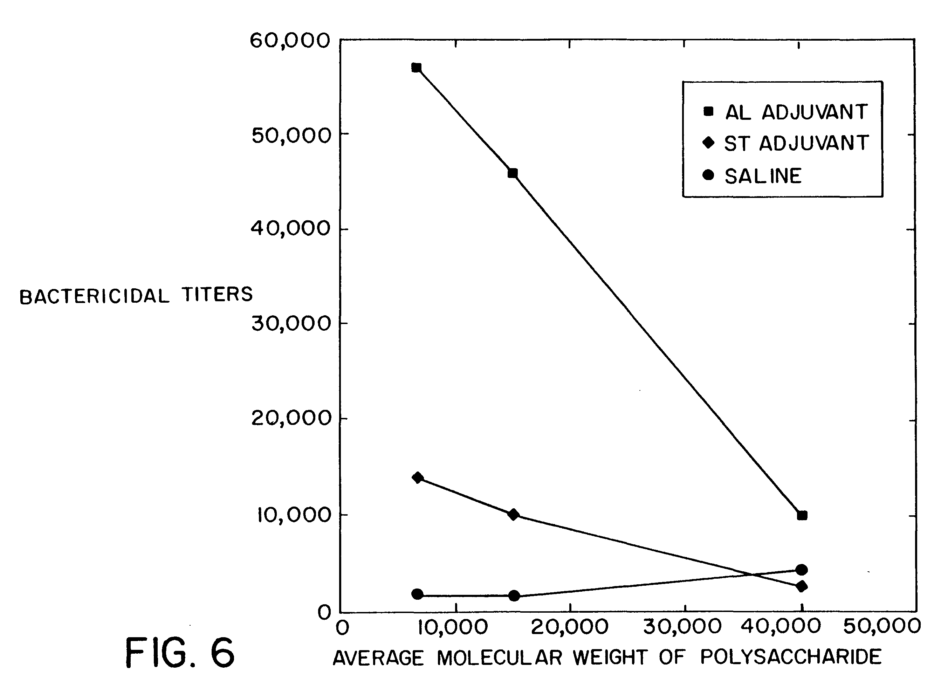

The immune responses to the group C meningococcal polysaccharide in the conjugate vaccines are expressed in terms of their ELISA titers. Each of the sera was analyzed by two methods. In the first the coating antigen [GCMP-Human Serum Albumin conjugate] contained O-acetyl-negative polysaccharide and in the second the coating antigen [GCMP-Bovine Serum Albumin conjugate] contained the O-acetyl-positive polysaccharide. The ELISA data is shown in figures 1, 2, 4 and 5. The sera from the controls which included free tetanus toxoid protein, O-acetyl positive polysaccharide and the O-acetyl negative polysaccharide did not give any detectable ELISA titers at the lowest dilutions analyzed (1/1,000). All of the sera also were analyzed for complement dependent bactericidal activities against N. meningitidis group C strain C11, an O-acetyl positive strain. The bactericidal titers shown in figures 3 and 6 are the sera dilutions yielding 50% survival. All of the sera from the controls showed no bactericidal activity at the lowest dilutions analyzed (1/1,000).

### Results and Discussion

The immune response to all of the conjugates prepared in this study was evaluated in three different vaccine formulations using both ELISA and the complement dependent bactericidal assay. The formulations include saline solutions of the conjugates or as adsorbed mixtures on aluminum hydroxide or stearyl tyrosine. The ELISAs on the sera from all of the conjugates show substantially higher immune responses when the conjugates were adsorbed on the aluminum hydroxide and lowest when they were injected as their saline solutions (see Figures 1, 2, 4 and 5). Conjugates complexed with stearyl tyrosine in all cases gave higher immune responses than the saline solutions but the immune responses also in all cases were several fold lower than for the aluminum hydroxide adsorbed conjugates. The bactericidal assay demonstrated the same trend for the three vaccine formulations (see Figures 3 and 6).

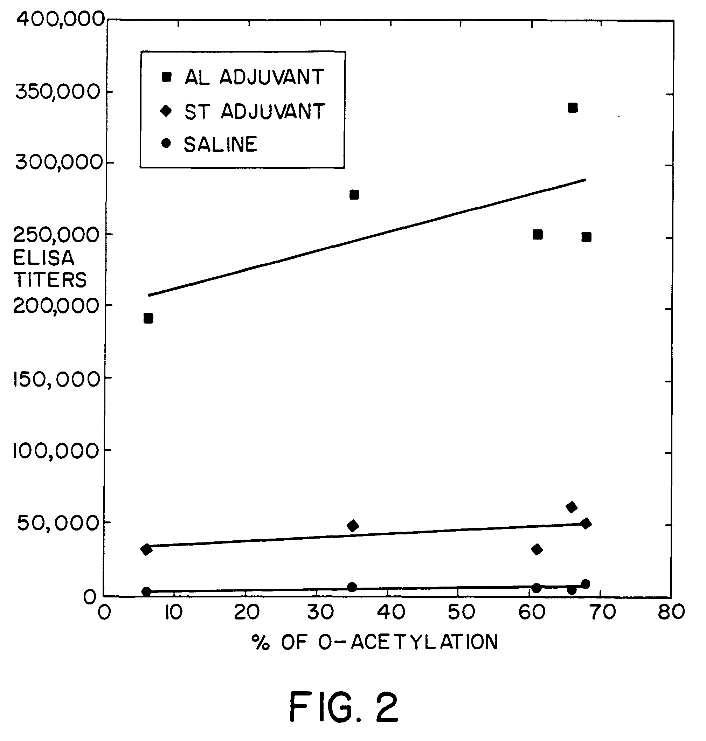

The ELISA titers for the GCMP-TT conjugates in which the average molecular weight of the conjugate polysaccharide is similar (12,000-14,800, see Table 1) are shown in Figures 1 and 2. In Figure 1 the ELISA titers were obtained with the coating antigen (GCMP-HSA) containing the O-acetyl-negative polysaccharide. With this antigen there is an inverse relationship between ELISA titers and the extent of O-acetylation of the conjugate polysaccharide such that highest titers have been obtained for the least O-acetylated polysaccharide. A completely different pattern is observed in Figure 2 in which are shown the titers obtained with the coating antigen (GCMP-BSA) containing the O-acetyl-positive polysaccharide. In this case the ELISA titers increase as the O-acetyl content increases, however, the differences in titers between the conjugates containing the least amount of O-acetylation and the most is not as large as was observed using the O-acetyl-negative coating antigen (see Figure 1). The relationship between the degree of O-acetylation and complement dependent bactericidal activity against the meningococcal group C strain C11 (an O-acetyl-positive strain) is shown in Figure 3. There is a marked decrease in bactericidal activity as the degree of O-acetylation increases. The bactericidal activity thus correlates closely with the ELISA titers obtained with the O-acetyl-negative coating antigen (see Figure 1). For the conjugates adsorbed on aluminum hydroxide, in both the ELISA and the bactericidal assay approximately a three fold decrease in titers is observed as the degree of O-acetylation increases from 5 to 70 percent. As shown in Figures 1-3, the three formulations of the vaccines show the same trends in each of the three assays. Also the relationships shown in Figures 1-3 have been observed in repeated analysis of the sera.

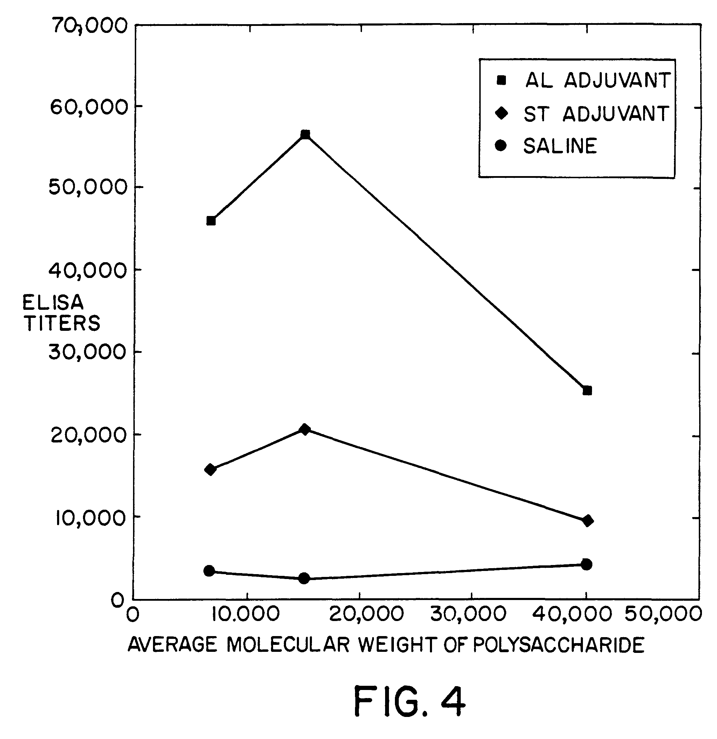

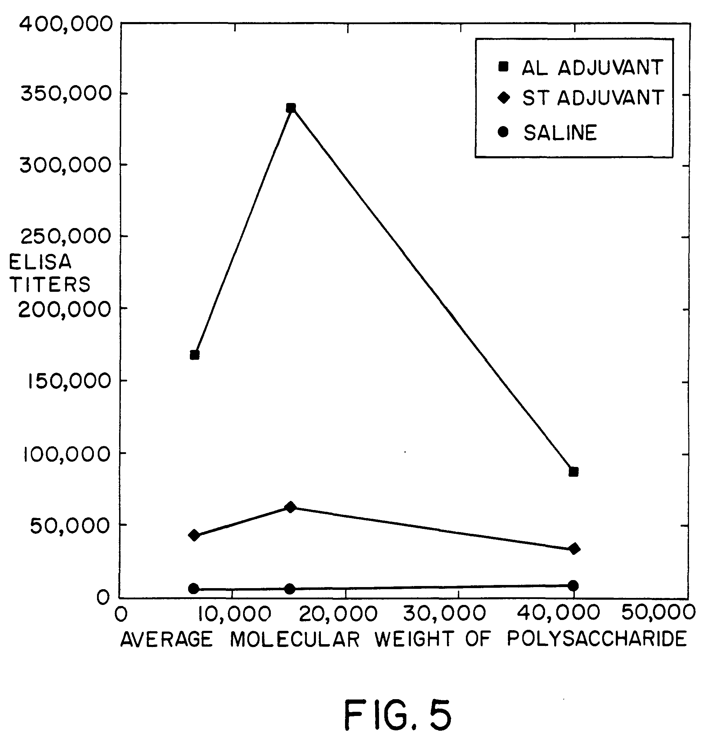

In addition to degree of O-acetylation and the type of formulation, the size of the polysaccharide in the conjugate vaccines, has a major effect on the immune response. This is shown in Figures 4-6 for GCMP-TT conjugates in which the polysaccharide is 61-65 % O-acetylated. The same trends are observed in the two ELISAs (Figures 4 and 5). The highest ELISA titers are observed for the conjugate vaccines in which the polysaccharide has an average molecular weight of 12,000 daltons in the aluminum hydroxide and stearyl tyrosine formulations although the differences are especially large for aluminum hydroxide adsorbed conjugates. In contrast to the two ELISAs the bactericidal titers for the aluminum hydroxide and stearyl tyrosine formulations are highest for the 6,600 daltons polysaccharide and decrease as size of the polysaccharide increases (see Figure 6).

**Table 4**

| Average Molecular Weights (Av. Mol. Wt.) and Nature of the O-Acetylation in the Purified and Activated Polysaccharide Samples | | | | |
|---|---|---|---|---|
| | | % O-Acetylation | | |
| Polysaccharide Sample Number | Av. Mol. Wt. Daltons | 7-OAc | 8-OAc | (7 + 8)-OAc |
| 1 | 6,600 | 40 | 23 | 63 |
| 2 | 12,000 | 34 | 27 | 61 |
| 3 | 12,800 | 31 | 4 | 35 |
| 4 | 13,000 | 41 | 27 | 68 |
| 5 | 14,000 | - | - | 6 |
| 6 | 14,800 | 40 | 26 | 66 |
| 7 | 40,000 | 36 | 29 | 65 |
| % O-Acetylation is determined by high resolution NMR spectroscopy by comparing the integrals of the methine protons on positions 7 and 8 to those for the protons on position 3 of the sialic acid residues. | | | | |

**Table 5**

| Summary of the % Polysaccharide Contents in the GCMP-TT Conjugates | | |
|---|---|---|
| Conjugate Number | Polysaccharide Sample Number | % Polysaccharide in Conjugate |
| 1 | 1 | 35 |
| 2 | 2 | 34 |
| 3 | 3 | 41 |
| 4 | 4 | 48 |
| 5 | 5 | 38 |
| 6 | 6 | 40 |
| 7 | 7 | 30 |

## Claims

1. Immunogenic conjugate comprising group C meningococcal polysaccharide covalently coupled to polymeric carrier, including O-deacetylated O-acetyl-positive group C meningococcal polysaccharide or a fragment thereof, wherein the degree of deacetylation is greater than 80%, for use as a vaccine against N. meningitidis infection.

2. Immunogenic conjugate of claim 1, **characterized in that** the degree of deacetylation is 100%.

3. Use of an immunogenic conjugate comprising group C meningococcal polysaccharide covalently coupled to polymeric carrier, including O-deacetylated O-acetyl positive group C meningococcal polysaccharide or a fragment thereof, wherein the degree of deacetylation is greater than 80%, for the manufacture of a vaccine against N. meningitidis infection.

4. Use of an immunogenic conjugate in accordance with claim 3, **characterized in that** the degree of deacetylation is 100%.

5. A process for the manufacture of immunogenic conjugate for the preparation of a vaccine against N. meningitidis infection, comprising activation of group C meningococcal polysaccharide or fragment thereof for coupling and coupling activated polysaccharide or fragment thereof with a carrier molecule, **characterized in that** before the activation greater than 80% acetyl groups of the polysaccharide or fragment thereof are deacetylated.

6. The process of claim 5, **characterized in that** 100% acetyl groups are deacetylated.

7. The process of claim 5 or 6, **characterized in that** the deacetylation is carried out under mild basic conditions in aqueous medium.

8. The process of claim 7, **characterized in that** the deacetylation is carried out in 0,01 N to 0,5 N ammonium hydroxide or sodium hydroxide or potassium hydroxide at a temperature of 0 to 50°C, preferably 25°C.

9. The process of claim 8, **characterized in that** deacetylation is carried out for 1 to 25 hours, preferably for 16 hours, in 0,1 N NaOH.

10. A vaccine against N. meningitidis infection, comprising a pharmaceutically acceptable injectable excipient and optionally physiologically acceptable adjuvant, **characterized in that** it contains as immunogenic component an immunogenic amount of conjugate of polymeric carrier covalently coupled with an O-deacetylated O-acetyl-positive group C meningococcal polysaccharide or fragment thereof, O-deacetylated in greater than 80%.

11. The vaccine according to claim 10, **characterized in that** it contains said polysaccharide or fragment thereof 100% deacetylated.

## Patentansprüche

1. Immunogenes Konjugat, umfassend Gruppe-C-Meningokokken-Polysaccharid in kovalenter Bindung an einen polymeren Träger, enthaltend O-desacetyliertes O-Acetyl-positives Gruppe-C-Meningokokken-Polysaccharid oder ein Fragment davon, wobei der Grad der Desacetylierung mehr als 80% beträgt, zur Verwendung als eine Vakzine gegen N. meningitidis-Infektion.

2. Immunogenes Konjugat nach Anspruch 1, **dadurch gekennzeichnet, dass** der Grad der Desacetylierung 100% beträgt.

3. Verwendung eines immunogenen Konjugats, umfassend Gruppe-C-Meningokokken-Polysaccharid in kovalenter Bindung an einen polymeren Träger, enthaltend O-desacetyliertes O-Acetyl-positives Gruppe-C-Meningokokken-Polysaccharid oder ein Fragment davon, wobei der Grad der Desacetylierung mehr als 80% beträgt, für die Herstellung einer Vakzine gegen N. meningitidis-Infektion.

4. Verwendung eines immunogenen Konjugats nach Anspruch 3, **dadurch gekennzeichnet, dass** der Grad der Desacetylierung 100% beträgt.

5. Verfahren zur Herstellung eines immunogenen Konjugats für die Präparation einer Vakzine gegen N. meningitidis-Infektion, umfassend die Aktivierung von Gruppe-C-Meningokokken-Polysaccharid oder eines Fragments davon zur Bindung, und die Bindung des aktivierten Polysaccharids oder Fragments davon an ein Träger-Molekül, **dadurch gekennzeichnet, dass** vor der Aktivierung mehr als 80% der Acetylgruppen des Polysaccharids oder Fragments davon desacetyliert werden.

6. Verfahren nach Anspruch 5, **dadurch gekennzeichnet, dass** 100% der Acetylgruppen desacetyliert sind.

7. Verfahren nach Anspruch 5 oder 6, **dadurch gekennzeichnet, dass** die Desacetylierung unter leicht basischen Bedingungen in wässrigem Medium vorgenommen wird.

8. Verfahren nach Anspruch 7, **dadurch gekennzeichnet, dass** die Desacetylierung in 0,01 N bis 0,5 N Ammoniumhydroxid oder Natriumhydroxid oder Kaliumhydroxid bei einer Temperatur von 0 bis 50°C, vorzugsweise 25°C, vorgenommen wird.

9. Verfahren nach Anspruch 8, **dadurch gekennzeichnet, dass** die Desacetylierung über 1 bis 25 Stunden, bevorzugt 16 Stunden hinweg in 0,1 N NaOH vorgenommen wird.

10. Vakzine gegen N. meningitidis-Infektion, umfassend einen pharmazeutisch geeigneten injizierbaren Arzneimittelträger und wahlweise ein physiologisch geeignetes Adjuvans, **dadurch gekennzeichnet, dass** es als immunogene Komponente eine immunogene Menge an Konjugat aus einem polymeren Träger in kovalenter Bindung an ein O-desacetyliertes O-Acetyl-positives Gruppe-C-Meningokokken-Polysaccharid oder Fragment davon enthält, das zu mehr als 80% O-desacetyliert ist.

11. Vakzine nach Anspruch 10, **dadurch gekennzeichnet, dass** es das Polysaccharid oder Fragment davon zu 100% desacetyliert enthält.

## Revendications

1. Conjugué immunogène comprenant un polysaccharide du méningocoque du groupe C couplé de manière covalente à un porteur polymérique, comprenant un polysaccharide du méningocoque du groupe C O-acétyle positif O-désacétylé ou un fragment de celui-ci, où le degré de désacétylation est plus grand que 80%, pour une utilisation comme vaccin contre une infection par N. meningitidis.

2. Conjugué immunogène de la revendication 1, **caractérisé en ce que** le degré de désacétylation est de 100%.

3. Utilisation d'un conjugué immunogène comprenant un polysaccharide du méningocoque du groupe C couplé de manière covalente à un support polymérique comprenant un polysaccharide du méningocoque du groupe C O-acétyle positif O-désacétylé ou un fragement de celui-ci, où le degré de désacétylation est plus grand que 80%, pour la fabrication d'un vaccin contre une infection par N. meningitidis.

4. Utilisation d'un conjugué immunogène selon la revendication 3, **caractérisé en ce que** le degré de désacétylation est de 100%.

5. Procédé pour la fabrication d'un conjugué immunogène pour la préparation d'un vaccin contre une infection par N. meningitidis, comprenant l'activation d'un polysaccharide du méningocoque du groupe C ou d'un fragment de celui-ci pour un couplage et le couplage du polysaccharide activé ou son fragment avec une molécule de support, **caractérisé en ce qu'**avant l'activation, plus de 80% des groupes acétyles du polysaccharide ou son fragment sont désacétylés.

6. Procédé de la revendication 5, **caractérisé en ce que** 100% des groupes acétyles sont désacétylés.

7. Procédé de la revendication 5 ou 6, **caractérisé en ce que** la désacétylation est effectuée en conditions modérément basiques dans un milieu aqueux.

8. Procédé de la revendication 7, **caractérisé en ce que** la désacétylation est effectuée dans de l'hydroxyde d'ammonium ou de l'hydroxyde de sodium ou de l'hydroxyde de potassium 0,01 N à 0,5 N à une température de 0 à 50°C, de préférence de 25°C.

9. Procédé de la revendication 8, **caractérisé en ce que** la désacétylation est effectuée pendant 1 à 25 heures, de préférence pendant 16 heures, dans NaOH 0,1 N.

10. Vaccin contre une infection par N meningitidis, comprenant un excipient injectable pharmaceutiquement acceptable et un adjuvant optionnel physiologiquement acceptable, **caractérisé en ce qu'**il contient, comme composant immunogène, une quantité immunogène d'un conjugué d'un support polymérique couplé de manière covalente avec un polysaccharide du méningocoque du groupe C O-acétyle positif O-désacétylé ou un fragment de celui-ci, O-désacétylé à plus de 80%.

11. Vaccin selon la revendication 10, **caractérisé en ce qu'**il contient ledit polysaccharide ou son fragment 100% désacétylé.
